# EUROPEAN PATENT SPECIFICATION

(11) **EP 4 289 357 B1**
(45) Date of publication and mention of the grant of the patent: **19.08.2026**
(21) Application number: 21924180.9
(22) Date of filing: 30.08.2021
(51) Int. Cl.: A61B 5/25, A61B 5/00

(54) **HUMAN BODY SIGNAL COLLECTION APPARATUS**
VORRICHTUNG ZUR ERFASSUNG VON SIGNALEN DES MENSCHLICHEN KÖRPERS
APPAREIL DE COLLECTE DE SIGNAUX CORPORELS HUMAINS

(30) Priority: 02.02.2021 CN 202110142469
(43) Date of publication of application: 13.12.2023
(73) Proprietor: Wuhan United Imaging Surgical Co., Ltd., Wuhan, Hubei 430206 (CN)
(72) Inventor: HE, Zhuobiao, Wuhan, Hubei 430206 (CN); TIAN, Jun, Wuhan, Hubei 430206 (CN); CHEN, Yifeng, Wuhan, Hubei 430206 (CN); LI, Dun Alex, Wuhan, Hubei 430206 (CN)
(74) Representative: Balder IP Law, S.L.
(86) International application number: PCT/CN2021/115352
(87) International publication number: WO 2022/166181

(56) References cited:
- EP-A1- 0 335 977
- EP-B1- 0 813 386
- WO-A1-2014/021886
- WO-A1-2015/188779
- CN-A- 102 488 517
- CN-A- 102 755 161
- CN-A- 107 510 447
- CN-A- 108 742 611
- CN-A- 110 338 783
- CN-A- 110 584 646
- CN-A- 112 274 157
- CN-U- 204 950 900
- JP-A- 2013 188 279
- US-A1- 2010 060 300
- US-A1- 2017 095 167

## Description

### TECHNICAL FIELD

The present application relates to the field of medical technology, in particular to a human-body-signal collection device.

### BACKGROUND

In the process of medical diagnosis, human body signals need to be detected usually by means of electrode-signal detecting instruments. The accuracy of signal detection affects the accuracy of a doctor's diagnosis.

However, in the process of collecting the detected human-body-signal, influenced by aging of an electrode itself, a relative movement between the electrode and skin, and a press on the skin, a baseline drift may occur in the detected human-body-signal. The frequency of an artifact signal caused by the baseline drift will cover the frequency of the detected human-body-signal, thus affecting accuracy of the detection.

In the related art, Pseudo-Noise Code (PN code) is a coded sequence composed of 0 and 1 with self-correlation properties similar to white noise, and used for anti-interference of spread spectrum in CDMA and WCDMA. Information of spread spectrum is distributed in a wider frequency band with a lower power spectrum density. When receiving and demodulating, the known PN code is used to correlate the received signal to demodulate to obtain user information. The PN code is also widely used in the field of satellite navigation and positioning. Each satellite has a unique PN code sequence generated by a complex pseudo-random code generator. These sequences have favorable properties in time and space, including high self-correlation and low cross-correlation, which helps receivers accurately identify and track specific satellite signals.

APN code synchronization process is divided into PN code capture (precise synchronization) and PN code tracking (fine synchronization). The PN code sequence capture refers to selecting and adjusting of a local spread spectrum PN sequence phase of a receiver when the receiver starts to receive the spread spectrum signal, so that the local spread spectrum PN sequence phase is basically consistent with the transmitted spread spectrum PN sequence phase, that is, the receiver captures the transmitted spread spectrum PN sequence phase, which is also known as a spread spectrum PN sequence synchronization.

When the receiving system is searching for synchronization, a code sequence generator of the receiving system operates at a rate different from that of the code sequence generator of the transmitter, causing two code sequences to slide against each other in phase until the two code sequences reach a consistent point, which is called a sliding correlation method. The received signal is multiplied by the local PN code and then integrated to obtain a cross-correlation value thereof, then the cross-correlation value is compared with a threshold value of a threshold detector to determine whether a useful signal has been captured. The correlation characteristics of the PN code sequence is used, and when two identical code sequences are in phase, the outputted correlation value is maximum. Once it is confirmed that the capture completes, the synchronization pulse of the instruction signal is captured to control a searching control clock to adjust the repetition frequency and phase of the PN code generated by the PN code generator, so that the PN code generated by the PN code generator is synchronized with the received signal.

The sliding correlation method above is one of the basic methods of correlating PN codes.

Document EP0335977A1 discloses a method of measuring biopotentials wherein an electrode is brought into contact with or is brought close to a living body in order to detect an electrical signal generated in the living body based on a potential induced on the electrode. Document US2010/060300A1 discloses a sensor system and several methods for the capacitive measurement of electromagnetic signals having a biological origin. Document WO2014/021886A1 discloses a system and method for determining at least one patient parameter using electrophysiological signals from the patient.

### SUMMARY

In view of this, the present invention is directed to a human-body-signal collection device as defined in the independent claim. Further aspects of the invention are defined in dependent claims.

The present application provides a human-body-signal collection device, including: a reference electrode, a first electrode and a second electrode, an adjusting and generating device, a first collection device, and a first feedback device. The reference electrode is attached to an area to be detected. The first electrode and the second electrode are adapted to be attached to a torso of the human body at intervals, the first electrode is configured to output a first signal, and the second electrode is configured to output a second signal. The adjusting and generating device is connected to the reference electrode and configured to output a reference signal to the reference electrode. The first collection device is connected to the first electrode and the second electrode, respectively. The first collection device is configured to collect the first signal and the second signal, and generate a first potential-difference signal according to the first signal and the second signal. The first feedback device is connected to the first collection device and the adjusting and generating device, respectively. The first feedback device is configured to collect the reference signal and the first potential-difference signal, generate a first feedback signal according to the reference signal and the first potential-difference signal, and use the first feedback signal to compensate for the first potential-difference signal, to eliminate an artifact in the first potential-difference signal.

In an embodiment, the first feedback signal includes a first sub-feedback signal. The first feedback device includes a first executing device and a first signal processing device. The first executing device is connected to the first collection device, and configured to receive the first potential-difference signal. The first signal processing device is connected to the adjusting and generating device, the first collection device, and the first executing device, respectively. The first signal processing device is configured to collect the reference signal and the first potential-difference signal, and separate a first reference drift signal, which corresponds to the reference signal, from the first potential-difference signal according to the reference signal. The first signal processing device is further configured to obtain the first sub-feedback signal according to the reference signal and the first reference drift signal, and output the first sub-feedback signal to the first executing device. The first executing device is configured to use the first sub-feedback signal to compensate for the first potential-difference signal, to eliminate a multiplicative artifact in the first potential-difference signal.

In an embodiment, the first feedback signal includes a second sub-feedback signal, and the first feedback device includes a second executing device.

The second executing device is connected to the first executing device and the first signal processing device, respectively. The second executing device is configured to receive the first potential-difference signal, in which the multiplicative artifact has been eliminated. The first signal processing device is further configured to obtain the second sub-feedback signal according to the reference signal and the first reference drift signal, and output the second sub-feedback signal to the second executing device. The second executing device is configured to use the second sub-feedback signal to compensate for the first potential-difference signal in which the multiplicative artifact has been eliminated, to eliminate the additive artifact in the first potential-difference signal.

In an embodiment, the first signal processing device includes a multiplicative-artifact analysis module, an additive-artifact analysis module, and a comprehensive analysis module.

The multiplicative-artifact analysis module is connected to the first collection device and the adjusting and generating device, respectively. The multiplicative-artifact analysis module is configured to, after correlating PN codes of the first potential-difference signal and of the reference signal, analyze a change of an amplitude and a change of a phase of the first potential-difference signal. The change of the amplitude and the change of the phase characterize a change of multiplicative noise.

The additive-artifact analysis module is connected to the multiplicative-artifact analysis module, and configured to demodulate a real part and an imaginary part of an impedance according to the change of the amplitude of the first potential-difference signal.

The comprehensive analysis module is connected to the additive-artifact analysis module, the multiplicative-artifact analysis module, the first executing device, and the second executing device, respectively. In an embodiment, the first collection device further includes a first amplifier, a first gain amplifier, a first filter, and a first analog-to-digital conversion circuit.

A first input terminal of the first amplifier is connected to the first electrode, a second input terminal of the first amplifier is connected to the second electrode. The first amplifier is configured to collect the first signal and the second signal, and generate the first potential-difference signal according to the first signal and the second signal. The first gain amplifier is connected to an output terminal of the first amplifier, and configured to implement a gain for the first potential-difference signal. The first filter is connected to the first gain amplifier, and configured to implement a filtering for the first potential-difference signal, for which the gain has been implemented.

The first analog-to-digital conversion circuit is connected to the first filter, the first executing device, and the first signal processing device, respectively, and configured to implement an analog-to-digital conversion for the first potential-difference signal, for which the filtering has been implemented.

In an embodiment, the first analog-to-digital conversion circuit is connected to the multiplicative-artifact analysis module. The first analog-to-digital conversion circuit is configured to output the first potential-difference signal, for which the analog-to-digital conversion is implemented, to the multiplicative-artifact analysis module.

In an embodiment, the human-body-signal collection device further includes a first sensing device. The first sensing device is connected to the first signal processing device. The first sensing device is configured to detect a first motion signal of the first electrode and output the first motion signal to the first signal processing device, and the first signal processing device is configured to judge whether the first signal processing device works or not according to the first motion signal. In an embodiment, when the first motion signal is greater than a preset value, the first signal processing device works.

In an embodiment, the human-body-signal collection device further includes a first bias adjustment device.

In an embodiment, the human-body-signal collection device further includes a third electrode, a second collection device, and a second feedback device. The third electrode is attached to the torso of the human body, and configured to output a third signal. The second collection device is connected to the second electrode and the third electrode, respectively, and configured to collect the second signal and the third signal, and generate a second potential-difference signal according to the second signal and the third signal.

The second feedback device is connected to the second collection device and the adjusting and generating device, respectively, and configured to collect the reference signal and the second potential-difference signal, and configured to generate a second feedback signal according to the reference signal and the second potential-difference signal, and further configured to use the second feedback signal to compensate for the second potential-difference signal, to eliminate an artifact in the second potential-difference signal. In an embodiment, the human-body-signal collection device further includes a switch. The switch includes a signal input terminal, a first output terminal, and a second output terminal. The signal input terminal is connected to an output terminal of the adjusting and generating device. The first output terminal is connected to the first electrode; and the second output terminal is connected to the third electrode.

In an embodiment, the human-body-signal collection device further includes a central control device. The central control device is connected to the first feedback device and the second feedback device, respectively. The central control device is configured to collect the first potential-difference signal and the second potential-difference signal, in which the artifacts are eliminated, and obtain detected signals based on the first potential-difference signal and the second potential-difference signal.

In an embodiment, the human-body-signal collection device further includes a power supply module. The power supply module includes a battery and a power management module. The battery is connected to the power management module. The power management module is connected to the adjusting and generating device, the first collection device, the first feedback device, the second collection device, and the second feedback device, respectively.

The present application provides a human-body-signal collection device, including a reference electrode, a first electrode and a second electrode, an adjusting and generating device, a first collection device, and a first feedback device. The reference electrode is attached to an area to be detected. The first electrode and the second electrode are attached to a torso of the human body at intervals, the first electrode is configured to output a first signal, and the second electrode is configured to output a second signal. The adjusting and generating device is connected to the reference electrode and configured to output a reference signal to the reference electrode. The first collection device is connected to the first electrode and the second electrode, respectively. The first collection device is configured to collect the first signal and the second signal, and generate a first potential-difference signal according to the first signal and the second signal. The first feedback device is connected to the first collection device and the adjusting and generating device, respectively. The first feedback device is configured to collect the reference signal and the first potential-difference signal, generate a first feedback signal according to the reference signal and the first potential-difference signal, and use the first feedback signal to compensate for the first potential-difference signal, to eliminate an artifact in the first potential-difference signal. The third executing device is connected to the first collection device, to offset capacitance between the human body and a reference ground.

In an embodiment, the reference electrode, the first electrode and the second electrode are wet electrodes.

In an embodiment, the first collection device further includes a first amplifier, a first gain amplifier, a first filter, and a first analog-to-digital conversion circuit.

A first input terminal of the first amplifier is connected to the first electrode, and a second input terminal of the first amplifier is connected to the second electrode. The first amplifier is configured to collect the first signal and the second signal, and generate the first potential-difference signal according to the first signal and the second signal. A first gain amplifier is connected to an output terminal of the first amplifier, and configured to implement a gain for the first potential-difference signal. The first filter is connected to the first gain amplifier, and configured to implement a filtering for the first potential-difference signal, for which the gain has been implemented.

The first analog-to-digital conversion circuit is connected to the first filter, the first executing device, and the first signal processing device, respectively, and configured to implement an analog-to-digital conversion for the first potential-difference signal, for which the filtering has been implemented.

In an embodiment, the first feedback signal includes a third sub-feedback signal. A first terminal of the third executing device is connected between the first electrode and the first input terminal of the first amplifier. A second terminal of the third executing device is connected between the output terminal of the first amplifier and the input terminal of the first gain amplifier, and a control terminal of the third executing device is connected to the first signal processing device. The first signal processing device is further configured to obtain a third sub-feedback signal according to the reference signal and the first reference drift signal, and output the third sub-feedback signal to the control terminal of the third executing device. The control terminal of the third executing device is configured to adjust a capacitance value of the third executing device according to the third sub-feedback signal, to offset the capacitance between the human body and the reference ground.

In an embodiment, the third executing device is connected in parallel with the first amplifier. The third executing device is configured to adjust the capacitance value of the third executing device according to the third sub-feedback signal, which can change impedance of the first collection device, thereby changing a proportion of an input impedance the first collection device, reducing an influence of an equivalent capacitance, and improving anti-interference ability of the detected signal.

In an embodiment, the third executing device is a variable capacitor.

In an embodiment, the human-body-signal collection device further includes a first bias adjustment device connected between the first terminal of the third executing device and the first electrode.

In an embodiment, a signal terminal of the first bias adjustment device is connected to the first feedback device. The first feedback device is configured to generate a voltage adjustment signal according to the reference signal and the first potential-difference signal, and configured to output the voltage adjustment signal to the first bias adjustment device. The first bias adjustment device is configured to adjust a bias voltage according to the voltage adjustment signal, to realize a compensation for a DC bias voltage at the input port of the first collection device.

The human-body-signal collection device provided in the embodiment of the present application includes: the reference electrode, the first electrode and the second electrode, an adjusting and generating device, the first collection device, and the first feedback device. The reference electrode is attached to an area to be detected. The first electrode and the second electrode are attached to the torso of the human body at intervals, the first electrode is configured to output the first signal, and the second electrode is configured to output the second signal. The adjusting and generating device is connected to the reference electrode and configured to output the reference signal to the reference electrode. The first collection device is connected to the first electrode and the second electrode, respectively. The first collection device is configured to collect the first signal and the second signal, and generate the first potential-difference signal according to the first signal and the second signal. The first feedback device is connected to the first collection device and the adjusting and generating device, respectively. The first feedback device is configured to collect the reference signal and the first potential-difference signal, generate the first feedback signal according to the reference signal and the first potential-difference signal, and use the first feedback signal to compensate for the first potential-difference signal, to eliminate an artifact in the first potential-difference signal.

In the human-body-signal collection device, by arranging the adjusting and generating device and the reference electrode, the adjusting and generating device outputs the reference signal to the reference electrode. The superposition of the reference signal and a signal of the to-be-detected area of the human body is outputted to the first feedback device. The first feedback device obtains the first feedback signal based on the reference signal and the first potential-difference signal. The first feedback signal may characterize the baseline change of the reference signal after the reference signal passes through the human body. The first feedback device uses the first feedback signal to compensate for the first potential-difference signal, so that the influence of the artifact caused by the baseline drift of the first potential-difference signal may be eliminated, thereby reducing the influence of the baseline drift on the detected signal, and improving the accuracy of the detected human-body-signal.

### BRIEF DESCRIPTION OF THE DRAWINGS

In order to illustrate the technical solutions of the embodiments of the present application or the prior art more clearly, the drawings needed for the description of the embodiments or the prior art will briefly be introduced. Obviously, the accompanying drawings in the following description are only embodiments of the present application, and those skilled in the art may also obtain other drawings according to the provided drawings without creative efforts.
FIG. 1 is a schematic circuit diagram illustrating a human-body-signal collection device according to an embodiment of the present application;
FIG. 2 is a schematic circuit diagram illustrating the human-body-signal collection device according to another embodiment of the present application;
FIG. 3 is a schematic circuit diagram illustrating the human-body-signal collection device according to another embodiment of the present application.

Reference signs:
human-body-signal collection device 10; reference electrode 210; first electrode 220; second electrode 230; adjusting and generating device 30; first collection device 40; first amplifier 410; first input terminal of first amplifier 411; second input terminal of first amplifier 412; output terminal of first amplifier 413; first gain amplifier 420; first filter 430; first analog-to-digital conversion circuit 440; first feedback device 50; first executing device 510; first signal processing device 520; second executing device 530; third executing device 540; first terminal of third actuator 541; second terminal of third actuator 542; control terminal of third actuator 543; first bias adjustment device 60; first sensing device 610; third electrode 240; second collection device 70; second amplifier 710; first input terminal of second amplifier 711; second input terminal of second amplifier 712; output terminal of second amplifier 713; second gain amplifier 720; second filter 730; second analog-to-digital conversion circuit 740; second feedback device 80; fourth executing device 810; second signal processing device 820; fifth executing device 830; sixth executing device 840; first terminal of sixth executing device 841; second terminal of sixth executing device 842; control terminal of sixth executing device 843; switch 90; signal input terminal 910; first output terminal 920; second output terminal 930; central control device 100; second bias adjustment device 600; second sensing device 620.

### DETAILED DESCRIPTION OF THE EMBODIMENTS

The technical solutions in the embodiments of the application will be clearly and completely described with reference to the drawings of the embodiments of the application. Apparently, the described embodiments are some but not all embodiments of the application.

In order to make the objectives, technical solutions, and advantages of the present application clearer and to be better understood, the present application will be further described in detail by means of the following embodiments combining with the accompanying drawings. It should be understood that the specific embodiments described herein are only used to explain the present application, but not intended to limit the present application.

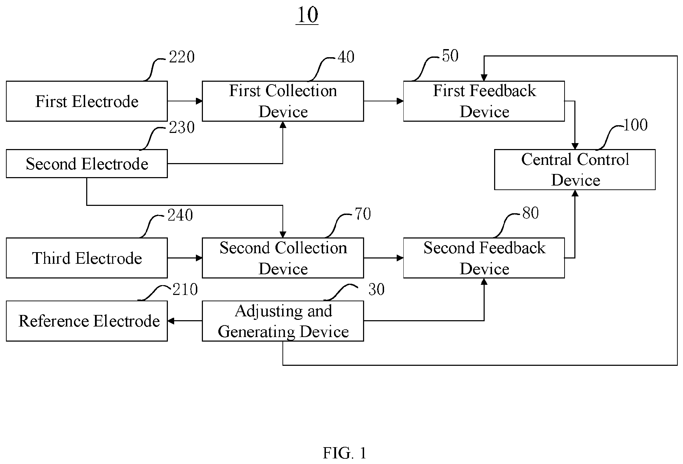

Referring to FIG. 1, the embodiment of the present application provides a human-body-signal collection device 10, including a reference electrode 210, a first electrode 220, a second electrode 230, an adjusting and generating device 30, a first collection device 40, and a first feedback device 50. The reference electrode 210 is attached to an area to be detected. The first electrode 220 and the second electrode 230 are attached to a torso of the human body at intervals. The first electrode 220 is configured to output a first signal. The second electrode 230 is configured to output a second signal. The adjusting and generating device 30 is connected to the reference electrode 210. The adjusting and generating device 30 is configured to output a reference signal to the reference electrode 210. The first collection device 40 is connected to the first electrode 220 and the second electrode 230, respectively. The first collection device 40 is configured to collect the first signal and the second signal, and generate a first potential-difference signal according to the first signal and the second signal. The first feedback device 50 is connected to the first collection device 40 and the adjusting and generating device 30, respectively. The first feedback device 50 is configured to collect the reference signal and the first potential-difference signal, generate a first feedback signal according to the reference signal and the first potential-difference signal, and use the first feedback signal to compensate for the first potential-difference signal, to eliminate an artifact in the first potential-difference signal.

In the human-body-signal collection device 10 provided in the embodiment of the present application, by arranging the adjusting and generating device 30 and the reference electrode 210, the adjusting and generating device 30 outputs the reference signal to the reference electrode 210. A superposition of the reference signal and a signal of the to-be-detected area of the human body is outputted to the first feedback device 50. The first feedback device 50 obtains the first feedback signal based on the reference signal and the first potential-difference signal. The first feedback signal may characterize a baseline change of the reference signal after the reference signal passes through the human body. The first feedback device 50 uses the first feedback signal to compensate for the first potential-difference signal, so that the influence of the artifact caused by the baseline drift of the first potential-difference signal may be eliminated, thereby reducing the influence of the baseline drift on the detected signal, and improving the accuracy of the detected human-body-signal.

The collected human-body-signal may be an electrocardiogram (ECG) signal, an electroencephalogram (EEG) signal, a respiratory signal, a muscle signal, and the like. The area to be detected may be heart, head, skin, muscle, or blood vessel. When the area to be detected is the heart, the human-body-signal collection device 10 is configured to detect cardiomyocytes, and the generated signal is the ECG signal. The ECG signal travels through the human body tissues to positions on the surface of the human body where the electrodes are attached. In an embodiment, the reference electrode 210 is arranged at a position corresponding to the heart. The position where the reference signal is added is approximately the same position where the ECG signal is generated, thereby ensuring that the transmission pathway of the reference signal is consistent with the transmission pathway of the ECG signal, that is, a transmission pathway from the heart to each of the electrodes. After the reference electrode 210 is arranged, the influences of the artifacts caused by motions and breathing, etc., on the ECG signal and the reference signal are approximately identical, therefore the artifact formed in the reference signal may be, to the greatest extent, consistent with the artifact superposed on the ECG.

The reference electrode 210, the first electrode 220, and the second electrode 230 may be wet electrodes. In an embodiment, the reference electrode 210, the first electrode 220 and the second electrode 230 are made of hydrogel materials.

The second electrode 230 is a common electrode, and the first electrode 220 is a detecting electrode. The first signal and the second signal are voltage signals. A difference between the voltage signals detected by the first electrode 220 and the second electrode 230 is the first potential-difference signal. The first potential-difference signal is also a voltage signal. The adjusting and generating device 30 is configured to generate a pseudo random signal. The frequency spectrum of the pseudo random signal is consistent with the frequency spectrums of the ECG signal, etc., and the amplitude of the pseudo random signal is much lower than the amplitudes of the ECG signal, etc., thereby reducing the influences of the first feedback signal on the collection of the ECG signal, and reducing an inaccuracy of a correction, which is caused by a great difference between contact impedances of the skin under different frequencies.

In an embodiment, if y denotes the first potential-difference signal generated by the first collection device 40, and x denotes the first potential-difference signal in an ideal state, then the relationship between y and x may be expressed as *y = A* × (*x + B*), where, *A* denotes a multiplicative artifact impact factor, and B denotes an additive artifact impact factor. The first feedback signal generated by the first feedback device 50 is configured to eliminate the multiplicative artifact impact factor and the additive artifact impact factor.

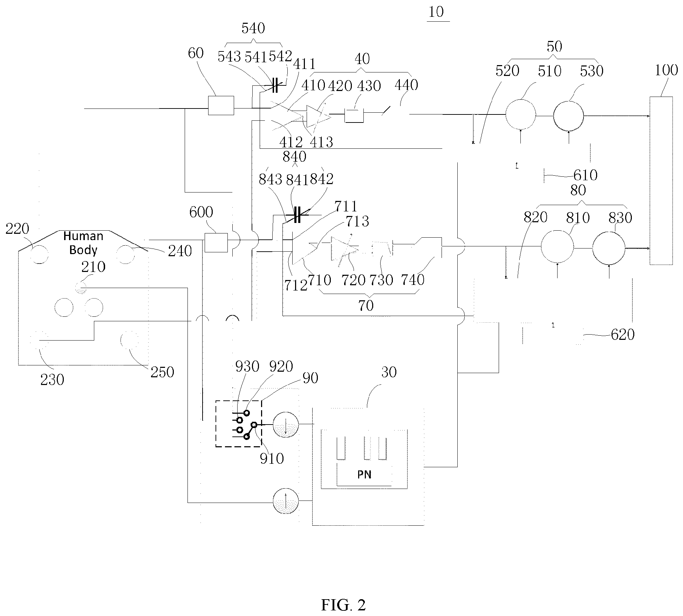

Referring to FIG. 2, in an embodiment, the first feedback signal includes a first sub-feedback signal. The first feedback device 50 includes a first executing device 510 and a first signal processing device 520.

The first executing device 510 is connected to the first collection device 40. The first executing device 510 is configured to receive the first potential-difference signal. The first signal processing device 520 is connected to the adjusting and generating device 30, the first collection device 40, and the first executing device 510, respectively. The first signal processing device 520 is configured to collect the reference signal and the first potential-difference signal, and separate a first reference drift signal, which corresponds to the reference signal, from the first potential-difference signal. The first signal processing device 520 is further configured to obtain the first sub-feedback signal according to the reference signal and the first reference drift signal, and output the first sub-feedback signal to the first executing device 510. The first executing device 510 is configured to use the first sub-feedback signal to compensate for the first potential-difference signal, to eliminate the multiplicative artifact in the first potential-difference signal.

The first sub-feedback signal may offset the multiplicative artifact of the first potential-difference signal. It may be understood that the multiplicative artifact factor may be eliminated by using the first sub-feedback signal to compensate for the first potential-difference signal.

In an embodiment, the first feedback signal includes a second sub-feedback signal, and the first feedback device 50 includes a second executing device 530. The second executing device 530 is connected to the first executing device 510 and the first signal processing device 520, respectively. The second executing device 530 is configured to receive the first potential-difference signal, in which the multiplicative artifact has been eliminated. The first signal processing device 520 is further configured to obtain the second sub-feedback signal according to the reference signal and the first reference drift signal, and output the second sub-feedback signal to the second executing device 530. The second executing device 530 is configured to use the second sub-feedback signal to compensate for the first potential-difference signal in which the multiplicative artifact has been eliminated, to eliminate the additive artifact in the first potential-difference signal.

The second sub-feedback signal may offset the additive artifact in the first potential-difference signal. It may be understood that the additive artifact factor may be eliminated by using the second sub-feedback signal to compensate for the first potential-difference signal.

It may be understood that the first signal processing device 520 simultaneously obtains the first sub-feedback signal and the second sub-feedback signal according to the reference signal and the first reference drift signal.

In an embodiment, the first signal processing device 520 includes a multiplicative-artifact analysis module, an additive-artifact analysis module and a comprehensive analysis module. The multiplicative-artifact analysis module is connected to the first collection device 40 and the adjusting and generating device 30, respectively. The multiplicative-artifact analysis module is configured to, after correlating PN codes of the first potential-difference signal and of the reference signal, analyze changes of the amplitude and the phase of the first potential-difference signal. The changes of the amplitude and phase characterize a change of the multiplicative noise. The additive-artifact analysis module is connected to the multiplicative-artifact analysis module. The additive-artifact analysis module is configured to demodulate a real part and an imaginary part of the impedance according to the change of the amplitude of the first potential-difference signal. The change of the imaginary part is strongly correlated with the additive artifact. The comprehensive analysis module is connected to the additive-artifact analysis module, the multiplicative-artifact analysis module, the first executing device 510, and the second executing device 530, respectively.

In an embodiment, the first collection device 40 further includes a first amplifier 410, a first gain amplifier 420, a first filter 430, and a first analog-to-digital conversion circuit 440. A first input terminal 411 of the first amplifier 410 is connected to the first electrode 220. A second input terminal 412 of the first amplifier 410 is connected to the second electrode 230. The first amplifier 410 is configured to collect the first signal and the second signal, and generate the first potential-difference signal according to the first signal and the second signal. The first gain amplifier 420 is connected to an output terminal 413 of the first amplifier 410. The first gain amplifier 420 is configured to implement a gain for the first potential-difference signal. The first filter 430 is connected to the first gain amplifier 420. The first filter 430 is configured to implement a filtering for the first potential-difference signal, for which the gain has been implemented. The first analog-to-digital conversion circuit 440 is connected to the first filter 430, the first executing device 510, and the first signal processing device 520, respectively. The first analog-to-digital conversion circuit 440 is configured to implement an analog-to-digital conversion for the first potential-difference signal, for which the filtering has been implemented.

The first amplifier 410 is configured to amplify the first signal and the second signal. The first potential-difference signal is a potential difference between the first signal and the second signal. The first gain amplifier 420 is configured to adjust the gain of the first potential-difference signal. The first analog-to-digital conversion circuit 440 is configured to change a type of the first potential-difference signal.

In an embodiment, the first analog-to-digital conversion circuit 440 is connected to the multiplicative-artifact analysis module. The first analog-to-digital conversion circuit 440 is configured to output the first potential-difference signal, for which the analog-to-digital conversion is implemented, to the multiplicative-artifact analysis module.

In an embodiment, the first feedback signal includes a third sub-feedback signal. The human-body-signal collection device 10 further includes a third executing device 540. A first terminal 541 of the third executing device 540 is connected between the first electrode 220 and the first input terminal 411 of the first amplifier 410. A second terminal 542 of the third executing device 540 is connected between the output terminal 413 of the first amplifier 410 and the input terminal of the first gain amplifier 420. A control terminal 543 of the third executing device 540 is connected to the first signal processing device 520. The first signal processing device 520 is further configured to obtain a third sub-feedback signal according to the reference signal and the first reference drift signal, and output the third sub-feedback signal to the control terminal 543 of the third executing device 540. The control terminal 543 of the third executing device 540 is configured to adjust a capacitance value of the third executing device 540 according to the third sub-feedback signal, to offset the capacitance between the human body and the ground.

An equivalent capacitance is formed between the electrodes and the reference ground. The equivalent capacitance causes a reduction of a high-frequency input impedance of the first collection device 40. The reduction of the input impedance worsens the anti-interference ability of the detected signal. The third executing device 540 is connected in parallel with the first amplifier 410. The third executing device 540 adjusts the capacitance value of the third executing device 540 according to the third sub-feedback signal, which can change the impedance of the first collection device 40, thereby changing a proportion of the input impedance of the first collection device 40, reducing the influence of the equivalent capacitance, and improving the anti-interference ability of the detected signal.

In an embodiment, the comprehensive analysis module is further connected to the third executing device 540. The comprehensive analysis module is also configured to import the change of the amplitude and a change of the imaginary part into an optimization algorithm, to obtain the first sub-feedback signal, the second sub-feedback signal, and the third sub-feedback signal. The comprehensive analysis module is further configured to output the third sub-feedback signal to the third executing device 540. The optimization algorithm may be an algorithm such as LMS.

The third executing device 540 may be a variable capacitor (compensating for negative capacitance). The capacitance value of the capacitor is equal to or approximate to the equivalent capacitance, so that an overall input impedance of the human-body-signal collection device 10 is the largest.

For a different detected individual, the equivalent capacitance between the electrodes and the human body is different. Therefore, a compensation method for an initial negative capacitance is used to increase the input impedance of the collection circuit, thereby improving the anti-interference performance. The capacitance value of the variable capacitor may be gradually fixed through multiple experiments. The experimental process includes testing the amplitude of the input signal by means of sweeping frequency, and when the amplitude of the signal in a key frequency band reaches a maximum, the capacitance value of the compensation capacitor is the optimal value.

In an embodiment, the human-body-signal collection device 10 further includes a first bias adjustment device 60. The first bias adjustment device 60 is connected between the first terminal 541 of the third executing device 540 and the first electrode 220.

A stretching of the skin, etc., may cause a change of a DC bias voltage. A polarization voltage (the DC bias voltage) generated by a contact between the electrode and the skin is related to a specific electrode, a position where the electrode is attached to the skin, an attaching time, and a motion, etc. The polarization voltage is as high as +/-300mV, and the DC voltage affects a common-mode rejection proportion and the input impedance of the first collection device 40, and a compensation is realized by independently adjusting the DC bias voltage at an input port of the first collection device 40.

In an embodiment, a signal terminal of the first bias adjustment device 60 is connected to the first feedback device 50. The first feedback device 50 is configured to generate a voltage adjustment signal according to the reference signal and the first potential-difference signal, and output the voltage adjustment signal to the first bias adjustment device 60. The first bias adjustment device 60 is configured to adjust the bias voltage according to the voltage adjustment signal, to realize the compensation for the DC bias voltage at the input port of the first collection device 40.

In an embodiment, the human-body-signal collection device 10 further includes a first sensing device 610. The first sensing device 610 is arranged proximate to the first electrode 220. The first sensing device 610 is connected to the first signal processing device 520. The first sensing device 610 is configured to detect a first motion signal of the first electrode 220 and output the first motion signal to the first signal processing device 520. The first signal processing device 520 is configured to judge whether it works or not according to the first motion signal.

The first sensing device 610 may be a motion sensing device. The first sensing device 610 may be a speed sensor, an acceleration sensor, or a displacement sensor, etc.

When the first motion signal is greater than a preset value, the first signal processing device 520 works. When the first motion signal is not greater than the preset value, the first signal processing device 520 does not work.

In an embodiment, at an initial period of the startup of the human-body-signal collection device 10, the first signal processing device 520 obtains the third sub-feedback signal according to the reference signal and the first reference drift signal, and the third executing device 540 adjusts the capacitance value according to the third sub-feedback signal. During the detection period of the human-body-signal collection device 10, the capacitance value of the third executing device 540 remains unchanged.

When the first motion signal is greater than the preset value, the first signal processing device 520 obtains the first sub-feedback signal and the second sub-feedback signal according to the reference signal and the first reference drift signal, so that the first executing device 510 and the second executing device 530 perform signal processing according to the feedback signals.

When the first motion signal is not greater than the preset value, the first signal processing device 520 does not process the reference signal and the first reference drift signal. The first executing device 510 and the second executing device 530 do not work.

In an embodiment, the human-body-signal collection device 10 further includes a third electrode 240, a second collection device 70, and a second feedback device 80. The third electrode 240 is attached to the torso of the human body. The third electrode 240 is configured to output a third signal. The second collection device 70 is connected to the second electrode 230 and the third electrode 240, respectively. The second collection device 70 is configured to collect the second signal and the third signal, and generate a second potential-difference signal according to the second signal and the third signal.

The second feedback device 80 is connected to the second collection device 70 and the adjusting and generating device 30, respectively. The second feedback device 80 is configured to collect the reference signal and the second potential-difference signal, and generate a second feedback signal according to the reference signal and the second potential-difference signal. The second feedback device 80 is further configured to use the second feedback signal to compensate for the second potential-difference signal, to eliminate the artifact in the second potential-difference signal.

Usually, the potential difference between the first potential-difference signal and the second potential-difference signal is used as reference information for diagnosing diseases, therefore, during the processing for the first potential-difference signal and for the second potential-difference signal, the related processing may not be performed for the second signal.

In the human-body-signal collection device 10, by arranging the adjusting and generating device 30 and the reference electrode 210, the adjusting and generating device 30 outputs the reference signal to the reference electrode 210. A superposition of the reference signal and the signal of the to-be-detected area of the human body is outputted to the second feedback device 80. The second feedback device 80 obtains the second feedback signal based on the reference signal and the second potential-difference signal. The second feedback signal may characterize the baseline change of the reference signal after the reference signal passes through the human body. The second feedback device 80 uses the second feedback signal to compensate for the second potential-difference signal, so that the influence of the artifact caused by the baseline drift of the second potential-difference signal may be eliminated, thereby reducing the influence of the baseline drift on the detected signal, and improving the accuracy of the detected human-body-signal.

In an embodiment, the human-body-signal collection device 10 may further include other detecting electrodes.

In an embodiment, the second feedback signal includes a fourth sub-feedback signal, and the second feedback device 80 includes a fourth executing device 810 and a second signal processing device 820.

The fourth executing device 810 is connected to the second collection device 70, and the fourth executing device 810 is configured to receive the second potential-difference signal.

The second signal processing device 820 is connected to the adjusting and generating device 30, the second collection device 70, and the fourth executing device 810. The second signal processing device 820 is configured to collect the reference signal and the second potential-difference signal, and separate a second reference, which corresponds to the reference signal, from the second potential-difference signal. The second signal processing device 820 is further configured to obtain the fourth sub-feedback signal according to the reference signal and the second reference drift signal, and output the fourth sub-feedback signal to the fourth executing device 810. The fourth executing device 810 is configured to use the fourth sub-feedback signal to compensate for the second potential-difference signal, to eliminate the multiplicative artifact in the second potential-difference signal.

The fourth sub-feedback signal may offset the multiplicative artifact of the second potential-difference signal. It may be understood that the multiplicative artifact factor may be eliminated by using the fourth sub-feedback signal to compensate for the second potential-difference signal.

In an embodiment, the second feedback signal includes a fifth sub-feedback signal. The second feedback device 80 includes a fifth executing device 830.

The fifth executing device 830 is connected to the fourth executing device 810 and the second signal processing device 820, respectively. The fifth executing device 830 is configured to receive the second potential-difference signal, in which the multiplicative artifact has been eliminated. The second signal processing device 820 is further configured to obtain the fifth sub-feedback signal according to the reference signal and the second reference drift signal, and output the fifth sub-feedback signal to the fifth executing device 830. The fifth executing device 830 is configured to use the fifth sub-feedback signal to compensate for the second potential-difference signal, in which the multiplicative artifact has been eliminated, to eliminate the additive artifact in the second potential-difference signal.

The fifth sub-feedback signal may offset the additive artifact in the second potential-difference signal. It may be understood that the additive artifact factor may be eliminated by using the fifth sub-feedback signal to compensate for the second potential- difference signal.

In an embodiment, the second collection device 70 further includes a second amplifier 710, a second gain amplifier 720, a second filter 730, and a second analog-to-digital conversion circuit 740. A first terminal 711 of the second amplifier 710 is connected to the third electrode 240. A second input terminal 712 of the second amplifier 710 is connected to the second electrode 230. The second amplifier 710 is configured to collect the third signal and the second signal, and generate the second potential-difference signal according to the third signal and the second signal. The second gain amplifier 720 is connected to an output terminal 713 of the second amplifier 710, and the second gain amplifier 720 is configured to implement a gain for the second potential-difference signal. The second filter 730 is connected to the second gain amplifier 720. The second filter 730 is configured to implement a filtering for the second potential-difference signal, for which the gain has been implemented. The second analog-to-digital conversion circuit 740 is connected to the second filter 730, the fourth executing device 810, and the second signal processing device 820, respectively. The second analog-to-digital conversion circuit 740 is configured to implement an analog-to-digital conversion for the second potential-difference signal, for which the filtering has been implemented.

In an embodiment, the second feedback signal includes a sixth sub-feedback signal. A sixth executing device 840 is further included. A first terminal 841 of the sixth executing device 840 is connected between the third electrode 240 and the first terminal 711 of the second amplifier 710. A second terminal 842 of the sixth executing device 840 is connected between the output terminal 713 of the second amplifier 710 and the input terminal of the second gain amplifier 720. The control terminal 843 of the sixth executing device 840 is connected to the second signal processing device 820. The second signal processing device 820 is further configured to obtain the sixth sub-feedback signal according to the reference signal and the second reference drift signal, and output the sixth sub-feedback signal to the control terminal 843 of the sixth executing device 840. The control terminal 843 of the sixth executing device 840 is configured to adjust a capacitance value of the sixth executing device 840 according to the sixth sub-feedback signal, to offset the capacitance between the human body and the ground.

The sixth executing device 840 is connected in parallel with the second amplifier 710. The sixth executing device 840 adjusts the capacitance value of the sixth executing device 840 according to the sixth sub-feedback signal, which can change the impedance of the second collection device 70, thereby changing the proportion of the input impedance of the second collection device 40, reducing the influence of the equivalent capacitance, and improving the anti-interference ability of the detected signal.

The sixth executing device 840 may be a variable capacitor (compensating for negative capacitance). The capacitance value of the capacitor is equal to or approximate to the equivalent capacitance, so that an overall input impedance of the human-body-signal collection device 10 is the largest.

In an embodiment, the human-body-signal collection device 10 further includes a second bias adjustment device 600. The second bias adjustment device 600 is connected between the first terminal 841 of the sixth executing device 840 and the third electrode 240. A compensation is realized by independently adjusting the DC bias voltage at an input port of the second collection device 70.

In an embodiment, the human-body-signal collection device 10 further includes a second sensing device 620. The second sensing device 620 is arranged proximate to the third electrode 240. The second sensing device 620 is connected to the second signal processing device 820. The second sensing device 620 is configured to detect a second motion signal of the third electrode 240, and output the second motion signal to the second signal processing device 820. The second signal processing device 820 is configured to judge whether it works or not according to the second motion signal.

In an embodiment, the human-body-signal collection device 10 further includes a switch 90. The switch 90 includes a signal input terminal 910, a first output terminal 920, and a second output terminal 930. The signal input terminal 910 is connected to the output terminal of the adjusting and generating device 30. The first output terminal 920 is connected to the first electrode 220. The second output terminal 930 is connected to the third electrode 240.

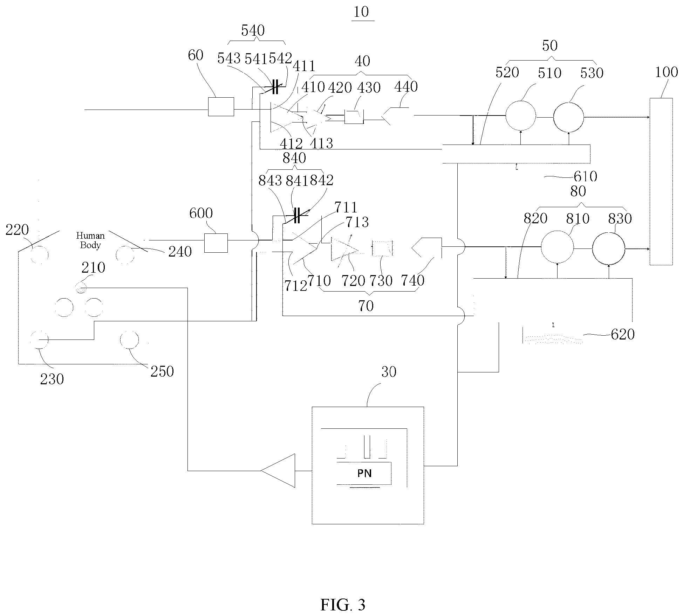

Referring to FIG. 3, if the human-body-signal collection device 10 is a current detection device, when the first output terminal 920 is connected to the signal input terminal 910, the first electrode 220 is connected to the adjusting and generating device 30 through the first output terminal 920. The adjusting and generating device 30, the reference electrode 210, the human body, and the first electrode 220 (a detecting electrode) form a closed loop. When the second output terminal 930 is connected to the signal input terminal 910, the third electrode 240 is connected to the adjusting and generating device 30 through the second output terminal 930. The adjusting and generating device 30, the reference electrode 210, the human body, and the third electrode 240 (a detecting electrode) form a closed loop. By switching the switch 90 to connect different electrodes for detection, it can be ensured that reference signals are consistent.

If the human-body-signal collection device 10 is a voltage detection device, there is no need to form a closed loop, what are needed are that the first collection device 40 needs to obtain an output voltage of the first electrode 220, and that the second collection device 70 needs to obtain an output voltage of the third electrode 240.

In an embodiment, the human-body-signal collection device 10 further includes a central control device 100. The central control device 100 is connected to the first feedback device 50 and the second feedback device 80, respectively. The central control device 100 is configured to collect the first potential-difference signal and the second potential-difference signal, in which the artifacts are eliminated, and obtain detected signals based on the first potential-difference signal and the second potential-difference signal.

In an embodiment, the human-body-signal collection device 10 further includes a power supply module. The power supply module includes a battery and a power management module. The battery is connected to the power management module. The power management module is connected to the adjusting and generating device 30, the first collection device 40, the first feedback device 50, the second collection device 70, and the second feedback device 80, respectively.

The battery may be a lithium battery or a battery having medium of any other chemical element. A rechargeable battery, or a battery of any other type, may be used to supply power. The battery is charged in a wired or wireless manner used by the power management module. The power management module may also record the power of the battery, and may stop supplying power when the power is too low, thereby ensuring a normal operation of the system.

In an embodiment, the human-body-signal collection device 10 further includes a clock. The clock is connected to the adjusting and generating device 30, the first collection device 40, the first feedback device 50, the second collection device 70, and the second feedback device 80, respectively. The clock is configured to regulate the working timing of the adjusting and generating device 30, of the first collection device 40, of the first feedback device 50, of the second collection device 70, and of the second feedback device 80.

Finally, it should also be noted that in the present disclosure, relational terms, such as "first" and "second" etc., are only used to distinguish one entity or operation from another, but do not necessarily require or imply that there are any such actual relationship or order for these entities or operations. Furthermore, the terms "include", "comprise", or any other variation thereof are intended to cover a non-exclusive inclusion, such that a process, a method, an article, or an apparatus including a series of elements not only includes those elements, but also includes elements not expressly listed, or includes other elements inherent in the process, in the method, in the article, or in the device. Without further limitations, an element defined by the phrase "including a..." does not exclude the presence of additional identical elements included in the process, in the method, in the article, or in the device.

Embodiments in the description are described in a progressive manner, and the description of each embodiment focuses on the difference of the embodiment from other embodiments, and the same and similar parts of all embodiments may be referred to each other.

## Claims

1. A human-body-signal collection device, comprising:
a reference electrode (210), attached to an area to be detected; and
a first electrode (220) and a second electrode (230), wherein the first electrode (220) and the second electrode (230) are adapted to be attached to a torso of the human body at intervals, the first electrode (220) is configured to output a first signal, and the second electrode (230) is configured to output a second signal;
an adjusting and generating device (30), connected to the reference electrode (210), and configured to output a reference signal to the reference electrode (210);
a first collection device (40), connected to the first electrode (220) and the second electrode (230), respectively, wherein the first collection device (40) is configured to collect the first signal and the second signal, and generate a first potential-difference signal according to the first signal and the second signal; and
a first feedback device (50), connected to the first collection device (40) and the adjusting and generating device (30), respectively, wherein the first feedback device (50) is configured to collect the reference signal and the first potential-difference signal, generate a first feedback signal according to the reference signal and the first potential-difference signal, and use the first feedback signal to compensate for the first potential-difference signal.

2. The human-body-signal collection device according to claim 1, wherein:
the first feedback signal comprises a first sub-feedback signal, and the first feedback device (50) comprises:
a first executing device (510), connected to the first collection device (40), and configured to receive the first potential-difference signal; and
a first signal processing device (520), connected to the adjusting and generating device (30), the first collection device (40), and the first executing device (510), respectively, wherein the first signal processing device (520) is configured to collect the reference signal and the first potential-difference signal, and separate a first reference drift signal, which corresponds to the reference signal, from the first potential-difference signal according to the reference signal; the first signal processing device (520) is further configured to obtain the first sub-feedback signal according to the reference signal and the first reference drift signal, and output the first sub-feedback signal to the first executing device (510); and the first executing device (510) is configured to use the first sub-feedback signal to compensate for the first potential-difference signal.

3. The human-body-signal collection device according to claim 2, wherein:
the first feedback signal comprises a second sub-feedback signal;
the first feedback device (50) comprises a second executing device (530);
the second executing device (530) is connected to the first executing device (510) and the first signal processing device (520), respectively; the second executing device (530) is configured to receive the first potential-difference signal, in which a multiplicative artifact has been eliminated; the first signal processing device (520) is further configured to obtain the second sub-feedback signal according to the reference signal and the first reference drift signal, and output the second sub-feedback signal to the second executing device (530); the second executing device (530) is configured to use the second sub-feedback signal to compensate for the first potential-difference signal in which the multiplicative artifact has been eliminated.

4. The human-body-signal collection device according to claim 3, wherein the first signal processing device comprises:
a multiplicative-artifact analysis module, connected to the first collection device (40) and the adjusting and generating device (30), respectively, wherein the multiplicative-artifact analysis module is configured to, after correlating PN codes of the first potential-difference signal and of the reference signal, analyze a change of an amplitude and a change of a phase of the first potential-difference signal, and the change of the amplitude and the change of the phase characterize a change of multiplicative noise;
an additive-artifact analysis module, connected to the multiplicative-artifact analysis module, and configured to demodulate a real part and an imaginary part of an impedance according to the change of the amplitude of the first potential-difference signal; and
a comprehensive analysis module, connected to the additive-artifact analysis module, the multiplicative-artifact analysis module, the first executing device (510), and the second executing device (530), respectively.

5. The human-body-signal collection device according to claim 4, wherein the first collection device (40) further comprises:
a first amplifier (410), wherein a first input terminal (411) of the first amplifier (410) is connected to the first electrode (220), a second input terminal (412) of the first amplifier (410) is connected to the second electrode (230), and the first amplifier (410) is configured to collect the first signal and the second signal, and generate the first potential-difference signal according to the first signal and the second signal;
a first gain amplifier (420), connected to an output terminal (413) of the first amplifier (410), and configured to implement a gain for the first potential-difference signal;
a first filter (430), connected to the first gain amplifier (420), and configured to implement a filtering for the first potential-difference signal, for which the gain has been implemented; and
a first analog-to-digital conversion circuit (440), connected to the first filter (430), the first executing device (510), and the first signal processing device (520), respectively, and configured to implement an analog-to-digital conversion for the first potential-difference signal, for which the filtering has been implemented;
preferably, the first analog-to-digital conversion circuit (440) is connected to the multiplicative-artifact analysis module; the first analog-to-digital conversion circuit (440) is configured to output the first potential-difference signal, for which the analog-to-digital conversion is implemented, to the multiplicative-artifact analysis module.

6. The human-body-signal collection device according to claim 2, further comprising a first sensing device (610), wherein:
the first sensing device (610) is connected to the first signal processing device (520); the first sensing device (610) is configured to detect a first motion signal of the first electrode (220) and output the first motion signal to the first signal processing device (520); and the first signal processing device (520) is configured to judge whether the first signal processing device (520) works or not according to the first motion signal;
preferably, when the first motion signal is greater than a preset value, the first signal processing device (520) works.

7. The human-body-signal collection device according to claim 1, further comprising:
a third electrode (240), attached to the torso of the human body, and configured to output a third signal;
a second collection device (70), connected to the second electrode (230) and the third electrode (240), respectively, and configured to collect the second signal and the third signal, and generate a second potential-difference signal according to the second signal and the third signal; and
a second feedback device (80), connected to the second collection device (70) and the adjusting and generating device (30), respectively, and configured to collect the reference signal and the second potential-difference signal, and configured to generate a second feedback signal according to the reference signal and the second potential-difference signal, and further configured to use the second feedback signal to compensate for the second potential-difference signal.

8. The human-body-signal collection device according to claim 7, further comprising:
a switch (90), wherein the switch (90) comprises a signal input terminal (910), a first output terminal (920), and a second output terminal (930); the signal input terminal (910) is connected to an output terminal of the adjusting and generating device (30); the first output terminal (920) is connected to the first electrode (220); and the second output terminal (930) is connected to the third electrode (240); or
a central control device (100), wherein the central control device (100) is connected to the first feedback device (50) and the second feedback device (80), respectively; and the central control device (100) is configured to collect the first potential-difference signal and the second potential-difference signal, in which artifacts are eliminated, and obtain detected signals based on the first potential-difference signal and the second potential-difference signal.

9. The human-body-signal collection device according to claim 7, wherein, the human-body-signal collection device (10) further comprises a power supply module; and
the power supply module comprises:
a power management module, connected to the adjusting and generating device (30), the first collection device (40), the first feedback device (50), the second collection device (70), and the second feedback device (80), respectively; and
a battery, connected to the power management module.

10. The human-body-signal collection device according to claim 1, further comprising:
a third executing device (540), connected to the first collection device (40), to offset capacitance between the human body and a reference ground.

11. The human-body-signal collection device according to claim 10, wherein the reference electrode (210), the first electrode (220) and the second electrode (230) are wet electrodes; or
the first collection device (40) further comprises:
a first amplifier (410), wherein a first input terminal (411) of the first amplifier (410) is connected to the first electrode (220), a second input terminal (412) of the first amplifier (410) is connected to the second electrode (230), and the first amplifier (410) is configured to collect the first signal and the second signal, and generate the first potential-difference signal according to the first signal and the second signal;
a first gain amplifier (420), connected to an output terminal (413) of the first amplifier (410), and configured to implement a gain for the first potential-difference signal;
a first filter (430), connected to the first gain amplifier (420), and configured to implement a filtering for the first potential-difference signal, for which the gain has been implemented; and
a first analog-to-digital conversion circuit (440), connected to the first filter (430), the first executing device (510), and the first signal processing device (520), respectively, and configured to implement an analog-to-digital conversion for the first potential-difference signal, for which the filtering has been implemented.

12. The human-body-signal collection device according to claim 11, wherein:
the first feedback signal comprises a third sub-feedback signal;
a first terminal (541) of the third executing device (540) is connected between the first electrode (220) and the first input terminal (411) of the first amplifier (410); a second terminal (542) of the third executing device (540) is connected between the output terminal (413) of the first amplifier (410) and the input terminal of the first gain amplifier (420); a control terminal (543) of the third executing device (540) is connected to the first signal processing device (520);
the first signal processing device (520) is further configured to obtain the third sub-feedback signal according to the reference signal and the first reference drift signal, and output the third sub-feedback signal to the control terminal (543) of the third executing device (540);
the control terminal (543) of the third executing device (540) is configured to adjust a capacitance value of the third executing device (540) according to the third sub-feedback signal.

13. The human-body-signal collection device according to claim 12, wherein the third executing device (540) is connected in parallel with the first amplifier (410); and the third executing device (540) is configured to adjust the capacitance value of the third executing device (540) according to the third sub-feedback signal;
preferably, the third executing device (540) is a variable capacitor.

14. The human-body-signal collection device according to claim 13, further comprising a first bias adjustment device (60) connected between the first terminal (541) of the third executing device (540) and the first electrode (220).

15. The human-body-signal collection device according to claim 14, wherein, a signal terminal of the first bias adjustment device (60) is connected to the first feedback device (50); the first feedback device (50) is configured to generate a voltage adjustment signal according to the reference signal and the first potential-difference signal, and configured to output the voltage adjustment signal to the first bias adjustment device (60); the first bias adjustment device (60) is configured to adjust a bias voltage according to the voltage adjustment signal, to realize a compensation for a DC bias voltage at the input port of the first collection device (40).

## Patentansprüche

1. Vorrichtung zur Erfassung von Signalen des menschlichen Körpers, umfassend:
eine Referenzelektrode (210), die an einem zu detektierenden Bereich angebracht ist; und
eine erste Elektrode (220) und eine zweite Elektrode (230), wobei die erste Elektrode (220) und die zweite Elektrode (230) angepasst sind, um in Intervallen an einem Rumpf des menschlichen Körpers angebracht zu werden, wobei die erste Elektrode (220) konfiguriert ist, um ein erstes Signal auszugeben, und die zweite Elektrode (230) konfiguriert ist, um ein zweites Signal auszugeben;
eine Anpassungs- und Erzeugungsvorrichtung (30), die mit der Referenzelektrode (210) verbunden und konfiguriert ist, um ein Referenzsignal an die Referenzelektrode (210) auszugeben;
eine erste Erfassungsvorrichtung (40), die jeweils mit der ersten Elektrode (220) und der zweiten Elektrode (230) verbunden ist, wobei die erste Erfassungsvorrichtung (40) konfiguriert ist, um das erste Signal und das zweite Signal zu erfassen und ein erstes Potenzialdifferenzsignal gemäß dem ersten Signal und dem zweiten Signal zu erzeugen; und
eine erste Feedback-Vorrichtung (50), die jeweils mit der ersten Erfassungsvorrichtung (40) und der Anpassungs- und Erzeugungsvorrichtung (30) verbunden ist, wobei die erste Feedback-Vorrichtung (50) konfiguriert ist, um das Referenzsignal und das erste Potenzialdifferenzsignal zu erfassen, ein erstes Feedbacksignal gemäß dem Referenzsignal und dem ersten Potenzialdifferenzsignal zu erzeugen und das erste Feedbacksignal zu verwenden, um das erste Potenzialdifferenzsignal zu kompensieren.

2. Vorrichtung zur Erfassung von Signalen des menschlichen Körpers nach Anspruch 1, wobei:
das erste Feedbacksignal ein erstes Unter-Feedbacksignal umfasst und die erste Feedback-Vorrichtung (50) umfasst:
eine erste Ausführungsvorrichtung (510), die mit der ersten Erfassungsvorrichtung (40) verbunden und konfiguriert ist, um das erste Potenzialdifferenzsignal zu empfangen; und
eine erste Signalverarbeitungsvorrichtung (520), die jeweils mit der Anpassungs- und Erzeugungsvorrichtung (30), der ersten Erfassungsvorrichtung (40) und der ersten Ausführungsvorrichtung (510) verbunden ist, wobei die erste Signalverarbeitungsvorrichtung (520) konfiguriert ist, um das Referenzsignal und das erste Potenzialdifferenzsignal zu erfassen und ein erstes Referenz-Driftsignal, welches dem Referenzsignal entspricht, aus dem ersten Potenzialdifferenzsignal gemäß dem Referenzsignal zu separieren;
wobei die erste Signalverarbeitungsvorrichtung (520) ferner konfiguriert ist, um das erste Unter-Feedbacksignal gemäß dem Referenzsignal und dem ersten Referenz-Driftsignal zu erhalten und das erste Unter-Feedbacksignal an die erste Ausführungsvorrichtung (510) auszugeben;
und die erste Ausführungsvorrichtung (510) konfiguriert ist, um das erste Unter-Feedbacksignal zu verwenden, um das erste Potenzialdifferenzsignal zu kompensieren.

3. Vorrichtung zur Erfassung von Signalen des menschlichen Körpers nach Anspruch 2, wobei:
das erste Feedbacksignal ein zweites Unter-Feedbacksignal umfasst;
die erste Feedback-Vorrichtung (50) eine zweite Ausführungsvorrichtung (530) umfasst;
die zweite Ausführungsvorrichtung (530) jeweils mit der ersten Ausführungsvorrichtung (510) und der ersten Signalverarbeitungsvorrichtung (520) verbunden ist;
die zweite Ausführungsvorrichtung (530) konfiguriert ist, um das erste Potenzialdifferenzsignal zu empfangen, in dem ein multiplikatives Artefakt eliminiert wurde;
die erste Signalverarbeitungsvorrichtung (520) ferner konfiguriert ist, um das zweite Unter-Feedbacksignal gemäß dem Referenzsignal und dem ersten Referenz-Driftsignal zu erhalten und das zweite Unter-Feedbacksignal an die zweite Ausführungsvorrichtung (530) auszugeben;
die zweite Ausführungsvorrichtung (530) konfiguriert ist, um das zweite Unter-Feedbacksignal zu verwenden, um das erste Potenzialdifferenzsignal, in dem das multiplikative Artefakt eliminiert wurde, zu kompensieren.

4. Vorrichtung zur Erfassung von Signalen des menschlichen Körpers nach Anspruch 3, wobei die erste Signalverarbeitungsvorrichtung umfasst:
ein Analysemodul für multiplikative Artefakte, das jeweils mit der ersten Erfassungsvorrichtung (40) und der Anpassungs- und Erzeugungsvorrichtung (30) verbunden ist, wobei das Analysemodul für multiplikative Artefakte konfiguriert ist, um nach dem Korrelieren von PN-Codes des ersten Potenzialdifferenzsignals und des Referenzsignals eine Änderung einer Amplitude und eine Änderung einer Phase des ersten Potenzialdifferenzsignals zu analysieren, wobei die Änderung der Amplitude und die Änderung der Phase eine Änderung des multiplikativen Rauschens charakterisieren;
ein Analysemodul für additive Artefakte, das mit dem Analysemodul für multiplikative Artefakte verbunden und konfiguriert ist, um einen Realteil und einen Imaginärteil einer Impedanz gemäß der Änderung der Amplitude des ersten Potenzialdifferenzsignals zu demodulieren; und
ein umfassendes Analysemodul, das jeweils mit dem Analysemodul für additive Artefakte, dem Analysemodul für multiplikative Artefakte, der ersten Ausführungsvorrichtung (510) und der zweiten Ausführungsvorrichtung (530) verbunden ist.

5. Vorrichtung zur Erfassung von Signalen des menschlichen Körpers nach Anspruch 4, wobei die erste Erfassungsvorrichtung (40) ferner umfasst:
einen ersten Verstärker (410), wobei ein erster Eingangsanschluss (411) des ersten Verstärkers (410) mit der ersten Elektrode (220) verbunden ist, ein zweiter Eingangsanschluss (412) des ersten Verstärkers (410) mit der zweiten Elektrode (230) verbunden ist, und der erste Verstärker (410) konfiguriert ist, um das erste Signal und das zweite Signal zu erfassen und das erste Potenzialdifferenzsignal gemäß dem ersten Signal und dem zweiten Signal zu erzeugen;
einen ersten Verstärkungsregelungs-Verstärker (420), der mit einem Ausgangsanschluss (413) des ersten Verstärkers (410) verbunden und konfiguriert ist, um eine Verstärkung für das erste Potenzialdifferenzsignal zu implementieren;
einen ersten Filter (430), der mit dem ersten Verstärkungsregelungs-Verstärker (420) verbunden und konfiguriert ist, um eine Filterung für das erste Potenzialdifferenzsignal, für welches die Verstärkung implementiert wurde, durchzuführen; und
eine erste Analog-Digital-Wandlerschaltung (440), die jeweils mit dem ersten Filter (430), der ersten Ausführungsvorrichtung (510) und der ersten Signalverarbeitungsvorrichtung (520) verbunden und konfiguriert ist, um eine Analog-Digital-Wandlung für das erste Potenzialdifferenzsignal, für welches die Filterung implementiert wurde, durchzuführen;
vorzugsweise ist die erste Analog-Digital-Wandlerschaltung (440) mit dem Analysemodul für multiplikative Artefakte verbunden;
wobei die erste Analog-Digital-Wandlerschaltung (440) konfiguriert ist, um das erste Potenzialdifferenzsignal, für welches die Analog-Digital-Wandlung implementiert ist, an das Analysemodul für multiplikative Artefakte auszugeben.

6. Vorrichtung zur Erfassung von Signalen des menschlichen Körpers nach Anspruch 2, ferner umfassend eine erste Sensorvorrichtung (610), wobei:
die erste Sensorvorrichtung (610) mit der ersten Signalverarbeitungsvorrichtung (520) verbunden ist;
die erste Sensorvorrichtung (610) konfiguriert ist, um ein erstes Bewegungssignal der ersten Elektrode (220) zu detektieren und das erste Bewegungssignal an die erste Signalverarbeitungsvorrichtung (520) auszugeben; und
die erste Signalverarbeitungsvorrichtung (520) konfiguriert ist, um gemäß dem ersten Bewegungssignal zu beurteilen, ob die erste Signalverarbeitungsvorrichtung (520) arbeitet oder nicht;
vorzugsweise arbeitet die erste Signalverarbeitungsvorrichtung (520), wenn das erste Bewegungssignal größer als ein voreingestellter Wert ist.

7. Vorrichtung zur Erfassung von Signalen des menschlichen Körpers nach Anspruch 1, ferner umfassend:
eine dritte Elektrode (240), die am Rumpf des menschlichen Körpers angebracht und konfiguriert ist, um ein drittes Signal auszugeben;
eine zweite Erfassungsvorrichtung (70), die jeweils mit der zweiten Elektrode (230) und der dritten Elektrode (240) verbunden und konfiguriert ist, um das zweite Signal und das dritte Signal zu erfassen und ein zweites Potenzialdifferenzsignal gemäß dem zweiten Signal und dem dritten Signal zu erzeugen; und
eine zweite Feedback-Vorrichtung (80), die jeweils mit der zweiten Erfassungsvorrichtung (70) und der Anpassungs- und Erzeugungsvorrichtung (30) verbunden ist, und konfiguriert ist, um das Referenzsignal und das zweite Potenzialdifferenzsignal zu erfassen, und konfiguriert ist, um ein zweites Feedbacksignal gemäß dem Referenzsignal und dem zweiten Potenzialdifferenzsignal zu erzeugen, und ferner konfiguriert ist, um das zweite Feedbacksignal zu verwenden, um das zweite Potenzialdifferenzsignal zu kompensieren.

8. Vorrichtung zur Erfassung von Signalen des menschlichen Körpers nach Anspruch 7, ferner umfassend:
einen Schalter (90), wobei der Schalter (90) einen Signaleingangsanschluss (910), einen ersten Ausgangsanschluss (920) und einen zweiten Ausgangsanschluss (930) umfasst;
wobei der Signaleingangsanschluss (910) mit einem Ausgangsanschluss der Anpassungs- und Erzeugungsvorrichtung (30) verbunden ist;
der erste Ausgangsanschluss (920) mit der ersten Elektrode (220) verbunden ist; und
der zweite Ausgangsanschluss (930) mit der dritten Elektrode (240) verbunden ist; oder
eine zentrale Steuervorrichtung (100), wobei die zentrale Steuervorrichtung (100) jeweils mit der ersten Feedback-Vorrichtung (50) und der zweiten Feedback-Vorrichtung (80) verbunden ist; und
die zentrale Steuervorrichtung (100) konfiguriert ist, um das erste Potenzialdifferenzsignal und das zweite Potenzialdifferenzsignal, in denen Artefakte eliminiert sind, zu erfassen und detektierte Signale basierend auf dem ersten Potenzialdifferenzsignal und dem zweiten Potenzialdifferenzsignal zu erhalten.

9. Vorrichtung zur Erfassung von Signalen des menschlichen Körpers nach Anspruch 7, wobei die Vorrichtung zur Erfassung von Signalen des menschlichen Körpers (10) ferner ein Stromversorgungsmodul umfasst;
und das Stromversorgungsmodul umfasst:
ein Stromverwaltungsmodul, das jeweils mit der Anpassungs- und Erzeugungsvorrichtung (30), der ersten Erfassungsvorrichtung (40), der ersten Feedback-Vorrichtung (50), der zweiten Erfassungsvorrichtung (70) und der zweiten Feedback-Vorrichtung (80) verbunden ist; und
eine Batterie, die mit dem Stromverwaltungsmodul verbunden ist.

10. Vorrichtung zur Erfassung von Signalen des menschlichen Körpers nach Anspruch 1, ferner umfassend:
eine dritte Ausführungsvorrichtung (540), die mit der ersten Erfassungsvorrichtung (40) verbunden ist, um eine Kapazität zwischen dem menschlichen Körper und einer Referenzmasse auszugleichen.

11. Vorrichtung zur Erfassung von Signalen des menschlichen Körpers nach Anspruch 10, wobei die Referenzelektrode (210), die erste Elektrode (220) und die zweite Elektrode (230) nasse Elektroden sind; oder
die erste Erfassungsvorrichtung (40) ferner umfasst:
einen ersten Verstärker (410), wobei ein erster Eingangsanschluss (411) des ersten Verstärkers (410) mit der ersten Elektrode (220) verbunden ist, ein zweiter Eingangsanschluss (412) des ersten Verstärkers (410) mit der zweiten Elektrode (230) verbunden ist, und der erste Verstärker (410) konfiguriert ist, um das erste Signal und das zweite Signal zu erfassen und das erste Potenzialdifferenzsignal gemäß dem ersten Signal und dem zweiten Signal zu erzeugen;
einen ersten Verstärkungsregelungs-Verstärker (420), der mit einem Ausgangsanschluss (413) des ersten Verstärkers (410) verbunden und konfiguriert ist, um eine Verstärkung für das erste Potenzialdifferenzsignal zu implementieren;
einen ersten Filter (430), der mit dem ersten Verstärkungsregelungs-Verstärker (420) verbunden und konfiguriert ist, um eine Filterung für das erste Potenzialdifferenzsignal, für welches die Verstärkung implementiert wurde, durchzuführen; und
eine erste Analog-Digital-Wandlerschaltung (440), die jeweils mit dem ersten Filter (430), der ersten Ausführungsvorrichtung (510) und der ersten Signalverarbeitungsvorrichtung (520) verbunden und konfiguriert ist, um eine Analog-Digital-Wandlung für das erste Potenzialdifferenzsignal, für welches die Filterung implementiert wurde, durchzuführen.

12. Vorrichtung zur Erfassung von Signalen des menschlichen Körpers nach Anspruch 11, wobei:
das erste Feedbacksignal ein drittes Unter-Feedbacksignal umfasst;
ein erster Anschluss (541) der dritten Ausführungsvorrichtung (540) zwischen der ersten Elektrode (220) und dem ersten Eingangsanschluss (411) des ersten Verstärkers (410) verbunden ist;
ein zweiter Anschluss (542) der dritten Ausführungsvorrichtung (540) zwischen dem Ausgangsanschluss (413) des ersten Verstärkers (410) und dem Eingangsanschluss des ersten Verstärkungsregelungs-Verstärkers (420) verbunden ist;
ein Steueranschluss (543) der dritten Ausführungsvorrichtung (540) mit der ersten Signalverarbeitungsvorrichtung (520) verbunden ist;
die erste Signalverarbeitungsvorrichtung (520) ferner konfiguriert ist, um das dritte Unter-Feedbacksignal gemäß dem Referenzsignal und dem ersten Referenz-Driftsignal zu erhalten und das dritte Unter-Feedbacksignal an den Steueranschluss (543) der dritten Ausführungsvorrichtung (540) auszugeben;
der Steueranschluss (543) der dritten Ausführungsvorrichtung (540) konfiguriert ist, um einen Kapazitätswert der dritten Ausführungsvorrichtung (540) gemäß dem dritten Unter-Feedbacksignal anzupassen, um die Kapazität zwischen dem menschlichen Körper und der Referenzmasse auszugleichen.

13. Vorrichtung zur Erfassung von Signalen des menschlichen Körpers nach Anspruch 12, wobei die dritte Ausführungsvorrichtung (540) parallel zum ersten Verstärker (410) geschaltet ist; und
die dritte Ausführungsvorrichtung (540) konfiguriert ist, um den Kapazitätswert der dritten Ausführungsvorrichtung (540) gemäß dem dritten Unter-Feedbacksignal anzupassen;
vorzugsweise ist die dritte Ausführungsvorrichtung (540) ein regelbarer Kondensator.

14. Vorrichtung zur Erfassung von Signalen des menschlichen Körpers nach Anspruch 13, ferner umfassend eine erste Bias-Einstellvorrichtung (60), die zwischen dem ersten Anschluss (541) der dritten Ausführungsvorrichtung (540) und der ersten Elektrode (220) verbunden ist.

15. Vorrichtung zur Erfassung von Signalen des menschlichen Körpers nach Anspruch 14, wobei ein Signalanschluss der ersten Bias-Einstellvorrichtung (60) mit der ersten Feedback-Vorrichtung (50) verbunden ist;
wobei die erste Feedback-Vorrichtung (50) konfiguriert ist, um ein Spannungsanpassungssignal gemäß dem Referenzsignal und dem ersten Potenzialdifferenzsignal zu erzeugen, und konfiguriert ist, um das Spannungsanpassungssignal an die erste Bias-Einstellvorrichtung (60) auszugeben;
wobei die erste Bias-Einstellvorrichtung (60) konfiguriert ist, um eine Bias-Spannung gemäß dem Spannungsanpassungssignal anzupassen, um eine Kompensation für eine DC-Bias-Spannung am Eingangsport der ersten Erfassungsvorrichtung (40) zu realisieren.

## Revendications

1. Dispositif de collecte de signaux de corps humain, comprenant :
une électrode de référence (210) fixée à une zone à détecter ; et
une première électrode (220) et une deuxième électrode (230), dans lequel la première électrode (220) et la deuxième électrode (230) sont adaptées pour être fixées à intervalles à un torse du corps humain, la première électrode (220) est configurée pour émettre un premier signal, et la deuxième électrode (230) est configurée pour émettre un deuxième signal ;
un dispositif d'ajustement et de génération (30) connecté à l'électrode de référence (210) et configuré pour émettre un signal de référence vers l'électrode de référence (210) ;
un premier dispositif de collecte (40) connecté respectivement à la première électrode (220) et à la deuxième électrode (230), dans lequel le premier dispositif de collecte (40) est configuré pour collecter le premier signal et le deuxième signal et générer un premier signal de différence de potentiel selon le premier signal et le deuxième signal ; et
un premier dispositif de rétroaction (50) connecté respectivement au premier dispositif de collecte (40) et au dispositif d'ajustement et de génération (30), dans lequel le premier dispositif de rétroaction (50) est configuré pour collecter le signal de référence et le premier signal de différence de potentiel, générer un premier signal de rétroaction selon le signal de référence et le premier signal de différence de potentiel, et utiliser le premier signal de rétroaction pour compenser le premier signal de différence de potentiel.

2. Dispositif de collecte de signaux de corps humain selon la revendication 1, dans lequel :
le premier signal de rétroaction comprend un premier sous-signal de rétroaction, et le premier dispositif de rétroaction (50) comprend :
un premier dispositif d'exécution (510) connecté au premier dispositif de collecte (40) et configuré pour recevoir le premier signal de différence de potentiel ; et
un premier dispositif de traitement de signal (520) connecté respectivement au dispositif d'ajustement et de génération (30), au premier dispositif de collecte (40) et au premier dispositif d'exécution (510), dans lequel le premier dispositif de traitement de signal (520) est configuré pour collecter le signal de référence et le premier signal de différence de potentiel et séparer un premier signal de dérive de référence qui correspond au signal de référence du premier signal de différence de potentiel selon le signal de référence ; le premier dispositif de traitement de signal (520) est en outre configuré pour obtenir le premier sous-signal de rétroaction selon le signal de référence et le premier signal de dérive de référence et émettre le premier sous-signal de rétroaction vers le premier dispositif d'exécution (510) ; et le premier dispositif d'exécution (510) est configuré pour utiliser le premier sous-signal de rétroaction pour compenser le premier signal de différence de potentiel.

3. Dispositif de collecte de signaux de corps humain selon la revendication 2, dans lequel :
le premier signal de rétroaction comprend un deuxième sous-signal de rétroaction ;
le premier dispositif de rétroaction (50) comprend un deuxième dispositif d'exécution (530) ;
le deuxième dispositif d'exécution (530) est connecté respectivement au premier dispositif d'exécution (510) et au premier dispositif de traitement de signal (520) ; le deuxième dispositif d'exécution (530) est configuré pour recevoir le premier signal de différence de potentiel duquel un artefact multiplicatif a été éliminé ; le premier dispositif de traitement de signal (520) est en outre configuré pour obtenir le deuxième sous-signal de rétroaction selon le signal de référence et le premier signal de dérive de référence et émettre le deuxième sous-signal de rétroaction vers le deuxième dispositif d'exécution (530) ; le deuxième dispositif d'exécution (530) est configuré pour utiliser le deuxième sous-signal de rétroaction pour compenser le premier signal de différence de potentiel duquel l'artefact multiplicatif a été éliminé.

4. Dispositif de collecte de signaux de corps humain selon la revendication 3, dans lequel le premier dispositif de traitement de signal comprend :
un module d'analyse des artefacts multiplicatifs connecté respectivement au premier dispositif de collecte (40) et au dispositif d'ajustement et de génération (30), dans lequel le module d'analyse des artefacts multiplicatifs est configuré pour, après avoir corrélé les codes PN du premier signal de différence de potentiel et du signal de référence, analyser un changement d'une amplitude et un changement d'une phase du premier signal de différence de potentiel, et le changement de l'amplitude et le changement de la phase caractérisent un changement du bruit multiplicatif ;
un module d'analyse d'artefacts additifs connecté au module d'analyse des artefacts multiplicatifs et configuré pour démoduler une partie réelle et une partie imaginaire d'une impédance selon le changement de l'amplitude du premier signal de différence de potentiel ; et
un module d'analyse complète connecté respectivement au module d'analyse d'artefacts additifs, au module d'analyse des artefacts multiplicatifs, au premier dispositif d'exécution (510) et au deuxième dispositif d'exécution (530).

5. Dispositif de collecte de signaux de corps humain selon la revendication 4, dans lequel le premier dispositif de collecte (40) comprend en outre :
un premier amplificateur (410), dans lequel une première borne d'entrée (411) du premier amplificateur (410) est connectée à la première électrode (220), une deuxième borne d'entrée (412) du premier amplificateur (410) est connectée à la deuxième électrode (230), et le premier amplificateur (410) est configuré pour collecter le premier signal et le deuxième signal et générer le premier signal de différence de potentiel selon le premier signal et le deuxième signal ;
un premier amplificateur de gain (420) connecté à une borne de sortie (413) du premier amplificateur (410) et configuré pour appliquer un gain au premier signal de différence de potentiel ;
un premier filtre (430) connecté au premier amplificateur de gain (420) et configuré pour appliquer un filtrage au premier signal de différence de potentiel auquel le gain a été appliqué ; et
un premier circuit de conversion analogique-numérique (440) connecté respectivement au premier filtre (430), au premier dispositif d'exécution (510) et au premier dispositif de traitement de signal (520) et configuré pour appliquer une conversion analogique-numérique au premier signal de différence de potentiel auquel le filtrage a été appliqué ;
de préférence, le premier circuit de conversion analogique-numérique (440) est connecté au module d'analyse des artefacts multiplicatifs ; le premier circuit de conversion analogique-numérique (440) est configuré pour émettre le premier signal de différence de potentiel auquel la conversion analogique-numérique a été appliquée vers le module d'analyse des artefacts multiplicatifs.

6. Dispositif de collecte de signaux de corps humain selon la revendication 2, comprenant en outre un premier dispositif de détection (610), dans lequel :
le premier dispositif de détection (610) est connecté au premier dispositif de traitement de signal (520) ;
le premier dispositif de détection (610) est configuré pour détecter un premier signal de mouvement de la première électrode (220) et émettre le premier signal de mouvement vers le premier dispositif de traitement de signal (520) ; et le premier dispositif de traitement de signal (520) est configuré pour estimer si le premier dispositif de traitement de signal (520) fonctionne ou non selon le premier signal de mouvement ;
de préférence, lorsque le premier signal de mouvement est supérieur à une valeur prédéfinie, le premier dispositif de traitement de signal (520) fonctionne.

7. Dispositif de collecte de signaux de corps humain selon la revendication 1, comprenant en outre :
une troisième électrode (240) fixée au torse du corps humain et configurée pour émettre un troisième signal ;
un deuxième dispositif de collecte (70) connecté respectivement à la deuxième électrode (230) et à la troisième électrode (240) et configuré pour collecter le deuxième signal et le troisième signal et générer un deuxième signal de différence de potentiel selon le deuxième signal et le troisième signal ; et
un deuxième dispositif de rétroaction (80) connecté respectivement au deuxième dispositif de collecte (70) et au dispositif d'ajustement et de génération (30) et configuré pour collecter le signal de référence et le deuxième signal de différence de potentiel, et configuré pour générer un deuxième signal de rétroaction selon le signal de référence et le deuxième signal de différence de potentiel, et configuré en outre pour utiliser le deuxième signal de rétroaction pour compenser le deuxième signal de différence de potentiel.

8. Dispositif de collecte de signaux de corps humain selon la revendication 7, comprenant en outre :
un commutateur (90), dans lequel le commutateur (90) comprend une borne d'entrée de signal (910), une première borne de sortie (920) et une deuxième borne de sortie (930) ; la borne d'entrée de signal (910) est connectée à une borne de sortie du dispositif d'ajustement et de génération (30) ; la première borne de sortie (920) est connectée à la première électrode (220) ; et la deuxième borne de sortie (930) est connectée à la troisième électrode (240) ; ou
un dispositif de commande central (100), dans lequel le dispositif de commande central (100) est connecté respectivement au premier dispositif de rétroaction (50) et au deuxième dispositif de rétroaction (80) ; et le dispositif de commande central (100) est configuré pour collecter le premier signal de différence de potentiel et le deuxième signal de différence de potentiel desquels les artefacts sont éliminés et obtenir des signaux détectés sur la base du premier signal de différence de potentiel et du deuxième signal de différence de potentiel.

9. Dispositif de collecte de signaux de corps humain selon la revendication 7, dans lequel le dispositif de collecte de signaux de corps humain (10) comprend en outre un module d'alimentation électrique ; et
le module d'alimentation électrique comprend :
un module de gestion d'alimentation connecté respectivement au dispositif d'ajustement et de génération (30), au premier dispositif de collecte (40), au premier dispositif de rétroaction (50), au deuxième dispositif de collecte (70) et au deuxième dispositif de rétroaction (80) ; et
une batterie connectée au module de gestion d'alimentation.

10. Dispositif de collecte de signaux de corps humain selon la revendication 1, comprenant en outre :
un troisième dispositif d'exécution (540) connecté au premier dispositif de collecte (40) pour contrebalancer la capacité entre le corps humain et une masse de référence.

11. Dispositif de collecte de signaux de corps humain selon la revendication 10, dans lequel l'électrode de référence (210), la première électrode (220) et la deuxième électrode (230) sont des électrodes humides ; ou
le premier dispositif de collecte (40) comprend en outre :
un premier amplificateur (410), dans lequel une première borne d'entrée (411) du premier amplificateur (410) est connectée à la première électrode (220), une deuxième borne d'entrée (412) du premier amplificateur (410) est connectée à la deuxième électrode (230), et le premier amplificateur (410) est configuré pour collecter le premier signal et le deuxième signal et générer le premier signal de différence de potentiel selon le premier signal et le deuxième signal ;
un premier amplificateur de gain (420) connecté à une borne de sortie (413) du premier amplificateur (410) et configuré pour appliquer un gain au premier signal de différence de potentiel ;
un premier filtre (430) connecté au premier amplificateur de gain (420) et configuré pour appliquer un filtrage au premier signal de différence de potentiel auquel le gain a été appliqué ; et
un premier circuit de conversion analogique-numérique (440) connecté respectivement au premier filtre (430), au premier dispositif d'exécution (510) et au premier dispositif de traitement de signal (520) et configuré pour appliquer une conversion analogique-numérique au premier signal de différence de potentiel auquel le filtrage a été appliqué.

12. Dispositif de collecte de signaux de corps humain selon la revendication 11, dans lequel :
le premier signal de rétroaction comprend un troisième sous-signal de rétroaction ; une première borne (541) du troisième dispositif d'exécution (540) est connectée entre la première électrode (220) et la première borne d'entrée (411) du premier amplificateur (410) ; une deuxième borne (542) du troisième dispositif d'exécution (540) est connectée entre la borne de sortie (413) du premier amplificateur (410) et la borne d'entrée du premier amplificateur de gain (420) ; une borne de commande (543) du troisième dispositif d'exécution (540) est connectée au premier dispositif de traitement de signal (520) ;
le premier dispositif de traitement de signal (520) est en outre configuré pour obtenir le troisième sous-signal de rétroaction selon le signal de référence et le premier signal de dérive de référence et émettre le troisième sous-signal de rétroaction vers la borne de commande (543) du troisième dispositif d'exécution (540) ;
la borne de commande (543) du troisième dispositif d'exécution (540) est configurée pour ajuster une valeur de capacité du troisième dispositif d'exécution (540) selon le troisième sous-signal de rétroaction.

13. Dispositif de collecte de signaux de corps humain selon la revendication 12, dans lequel le troisième dispositif d'exécution (540) est connecté en parallèle au premier amplificateur (410) ; et le troisième dispositif d'exécution (540) est configuré pour ajuster la valeur de capacité du troisième dispositif d'exécution (540) selon le troisième sous-signal de rétroaction ;
de préférence, le troisième dispositif d'exécution (540) est un condensateur variable.

14. Dispositif de collecte de signaux de corps humain selon la revendication 13, comprenant en outre un premier dispositif d'ajustement de polarisation (60) connecté entre la première borne (541) du troisième dispositif d'exécution (540) et la première électrode (220).

15. Dispositif de collecte de signaux de corps humain selon la revendication 14, dans lequel une borne de signal du premier dispositif d'ajustement de polarisation (60) est connectée au premier dispositif de rétroaction (50) ; le premier dispositif de rétroaction (50) est configuré pour générer un signal d'ajustement de tension selon le signal de référence et le premier signal de différence de potentiel, et configuré pour émettre le signal d'ajustement de tension vers le premier dispositif d'ajustement de polarisation (60) ; le premier dispositif d'ajustement de polarisation (60) est configuré pour ajuster une tension de polarisation selon le signal d'ajustement de tension pour compenser une tension de polarisation continue au niveau du port d'entrée du premier dispositif de collecte (40).
